# EUROPEAN PATENT SPECIFICATION

(11) **EP 1 829 545 B1**
(45) Date of publication and mention of the grant of the patent: **02.05.2012**
(21) Application number: 05819874.8
(22) Date of filing: 26.12.2005
(51) Int. Cl.: A61K 31/5575, A61K 9/00

(54) **PROSTAGLANDIN F2 ALPHA DERIVATIVE CONTAINING PRODUCTS**
PROSTAGLANDIN F2 ALPHA DERIVAT ENTHALTENDE PRODUKTE
PRODUITS CONTENANT UN DERIVE DE PROSTAGLANDINE F2 ALPHA

(30) Priority: 24.12.2004 JP 2004374009
(43) Date of publication of application: 05.09.2007
(73) Proprietor: SANTEN PHARMACEUTICAL CO., LTD., Higashiyodogawa-ku, Osaka-shi, Osaka 533-8651 (JP)
(72) Inventor: KIMURA, Akio, SANTEN PHARMACEUTICAL CO., LTD., Osaka-shi, Osaka 5338651 (JP); YAMADA, Hiroshi, SANTEN PHARMACEUTICAL CO., LTD., Osaka-shi, Osaka 5338651 (JP); KADO, Takehiro, SANTEN PHARMACEUTICAL CO., LTD., Osaka-shi, Osaka 5338651 (JP)
(74) Representative: Müller-Boré & Partner Patentanwälte
(86) International application number: PCT/JP2005/023704
(87) International publication number: WO 2006/068266

(56) References cited:
- EP-A- 1 321 144
- WO-A-00/03736
- WO-A-02/22106
- JP-A- 2002 161 037
- JP-A- 2002 520 368
- JP-A- 2003 138 074

## Description

The present invention relates to a prostaglandin F2α derivative-containing product, wherein an aqueous liquid preparation containing a prostaglandin F2α derivative having at least a fluorine atom in its molecule is stored in a resin container made of a propylene/ethylene copolymer in which the ratio of a propylene component to an ethylene component is in the range of from 96.4/3.6 to 98.7/1.3 (propylene component/ethylene component), whereby a decrease in the content of the prostaglandin F2α derivative in the aqueous liquid preparation is inhibited.

It is known that a prostaglandin F2α derivative having one or two fluorine atoms in its molecule is useful as a therapeutic agent for glaucoma and ocular hypertension (JP-A-10-251225 and JP-A-11-71344). However, such a prostaglandin F2α derivative having at least a fluorine atom in its molecule is generally slightly soluble in water and has a property of being easily adsorbed on a container, therefore, it is necessary to improve the problems of solubility in water and adsorptivity on a container.

JP-A-2002-161037 describes an invention of improving the solubility in water of a prostaglandin derivative and the adsorptivity thereof on a resin container by adding a nonionic surfactant such as polysorbate 80 to an aqueous liquid preparation, however, a resin container for storing the aqueous liquid preparation itself has not been studied. On the other hand, JP-T-2002-520368 describes that an aqueous prostaglandin composition containing prostaglandin and a surfactant is more stabilized when it is stored in a polypropylene resin container with an isotactic structure or a syndiotactic structure than when it is stored in a polyethylene resin container and a decrease in the residual ratio thereof can be inhibited.

However, in terms of a material of the resin container, there is no report that the stabilization of the prostaglandin derivative is studied by altering the ratio of the respective monomer components of a propylene/ethylene copolymer.

In this context, WO 02/22106 A2 describes a stable method for storing a pharmaceutical composition containing a prostaglandin-like compound comprising the step of storing the composition in a propylene container. Further, EP 1 321 144 A1 describes a method for improving the solubility of prostaglandin derivatives in water and inhibiting their absorption to resinous containers, as well as the respective prostaglandin derivatives. Furthermore, JP-A-2003138074 describes medical stretch blow molded containers made of propylene-oc-olefin copolymers. Finally, WO 00/03736 A1 describes a pharmaceutical product comprising an aqueous prostaglandin formulation and a polypropylene container.

It is an important object to provide a resin container in which an aqueous liquid preparation containing a prostaglandin F2α derivative having at least a fluorine atom in its molecule can be stored stably over a long period of time.

The present inventors studied the inhibition of the decrease in the content of a prostaglandin F2α derivative having at least a fluorine atom in its molecule by storing an aqueous liquid preparation containing the prostaglandin F2α derivative in a resin container made of a propylene/ethylene copolymer having different ratios of monomer components, and as a result, they surprisingly found that the decrease in the content of the prostaglandin F2α derivative can be significantly inhibited in a resin container made of a copolymer having a specific component ratio, and thus the present invention has been accomplished.

That is, the present invention relates to:
(1) a prostaglandin F2α derivative-containing product, wherein an aqueous liquid preparation containing a prostaglandin F2α derivative having at least a fluorine atom in its molecule is stored in a resin container made of a propylene/ethylene copolymer in which the ratio of a propylene component to an ethylene component is in the range of from 96.4/3.6 to 98.7/1.3 (propylene component/ethylene component), whereby a decrease in the content of the prostaglandin F2α derivative in the aqueous liquid preparation is inhibited;
(2) the prostaglandin F2α derivative-containing product according to the above (1), wherein the prostaglandin F2α derivative having at least a fluorine atom in its molecule is a difluoro-prostaglandin F2 α derivative;
(3) the prostaglandin F2α derivative-containing product according to the above (1), characterized in that a nonionic surfactant is contained in the aqueous liquid preparation;
(4) the prostaglandin F2α derivative-containing product according to the above (3), wherein the nonionic surfactant is polysorbate 80;
(5) the prostaglandin F2α derivative-containing product according to any one of the above (1) to (4), wherein the aqueous liquid preparation is an eye drop; and
(6) a method of inhibiting a decrease in the content of a prostaglandin F2α derivative having at least a fluorine atom in its molecule in an aqueous liquid preparation, comprising storing the aqueous liquid preparation containing the prostaglandin F2α derivative in a resin container made of a propylene/ethylene copolymer in which the ratio of a propylene component to an ethylene component is in the range of from 96.4/3.6 to 98.7/1.3 (propylene component/ethylene component)

The prostaglandin F2α derivative having at least a fluorine atom in its molecule of the present invention is generally slightly soluble in water and has a property of being easily adsorbed on a container. The phrase "the prostaglandin F2α derivative having at least a fluorine atom in its molecule is slightly soluble in water" means that 1000 ml or more of water is required for dissolving 1 g of the prostaglandin F2α derivative having at least a fluorine atom in its molecule (the Japanese Pharmacopoeia, 13th edition, General Rule A-51 (1996)). The phrase "a property of being easily adsorbed on a container" means that when the prostaglandin derivative is prepared as an aqueous solution and stored in a container, the content thereof (the "content" means the amount of prostaglandin existing in the aqueous solution as being effectively dissolved therein relative to the amount of prostaglandin dissolved) significantly decreases.

The prostaglandin F2α derivative having at least a fluorine atom in its molecule (hereinafter referred to as "fluorine-containing prostaglandin") of the present invention is not particularly limited as long as it is a prostaglandin F2α derivative having one to several fluorine atoms in its molecule. Preferred examples thereof include prostaglandin F2α derivatives disclosed in JP-A-10-251225 and JP-A-11-713-44, more preferred examples thereof include difluoro- prostaglandin F2α derivatives disclosed in JP-A-11-71344, and particularly preferred examples thereof include difluoro-prostaglandin F2 α derivatives having two fluorine atoms at its 15-position disclosed in JP-A-11-71344. Particularly preferred specific examples thereof include 16-phenoxy-15-deoxy-15,15-difluoro-17,18,19,20-tetranor-prostaglandin F2α, 16-(3-chlorophenoxy)-15-deoxy-15,15-difluoro-17,18,19,20-tetranor-prostaglandin F2α, 16-phenoxy-15-deoxy- 15,15-difluoro-13,14-dihydro-17,18,19,20-tetranor-prostaglandin F2α, alkyl esters thereof and salts thereof. Specific examples of the alkyl ester include lower alkyl esters such as methyl esters, ethyl esters, propyl esters, isopropyl esters, tert-butyl esters, pentyl esters and hexyl esters.

As will be explained in detail in the section of storage stability test described later, when the aqueous liquid preparation containing the fluorine-containing prostaglandin is stored in a resin container made of a propylene/ethylene copolymer in which the ratio of a propylene component to an ethylene component is in the range of from 96.4/3.6 to 98.7/1.3 (propylene component/ethylene component), the prostaglandin is clearly more stabilized than when it is stored in a container made of a propylene homopolymer and the adsorption thereof on the wall of the container is inhibited.

The resin container of the present invention can be obtained by molding a propylene/ethylene copolymer in which the ratio of a propylene component to an ethylene component is in the range of from 96.4/3.6 to 98.7/1.3 (propylene component/ethylene component). A molding method is exemplified by injection blow molding. The propylene/ethylene copolymer having a monomer component ratio as described above may be a copolymer obtained by any polymerization method. A preferred copolymer is, for example, a random copolymer obtained by random copolymerization of propylene and ethylene.

In the present invention, a nonionic surfactant may be added to the aqueous liquid preparation. The nonionic
surfactant is useful for inhibiting a decrease in the content of the fluorine-containing prostaglandin in the aqueous liquid preparation by improving the solubility in water of the fluorine-containing prostaglandin. Specific examples of the nonionic surfactant include polyoxyethylene fatty acid esters such as polysorbate 80 [polyoxyethylene sorbitan monooleate], polysorbate 60 [polyoxyethylene sorbitan monostearate], polysorbate 40 [polyoxyethylene sorbitan monopalmitate], polyoxyethylene sorbitan monolaurate, polyoxyethylene sorbitan trioleate and polysorbate 65 [polyoxyethylene sorbitan tristearate]; polyoxyethylene polyoxypropylene glycols such as polyoxyethylene (160) polyoxypropylene (30) glycol [Pluronic F68], polyoxyethylene (42) polyoxypropylene (67) glycol [Pluronic P123], polyoxyethylene (54) polyoxypropylene (39) glycol [Pluronic P85], polyoxyethylene (196) polyoxypropylene (67) glycol [Pluronic F127] and polyoxyethylene (20) polyoxypropylene (20) glycol [Pluronic L-44]; polyoxyl 40 stearate, sucrose fatty acid esters and the like. Preferred examples thereof include polysorbate 80 [polyoxyethylene sorbitan monooleate], polyoxyl 40 stearate and the like. These nonionic surfactants can be used alone or in combination of two or more of them. A particularly preferred nonionic surfactant is exemplified by polysorbate 80 [polyoxyethylene sorbitan monooleate], which is widely used as an additive for an eye drop.

It is preferred that in the prostaglandin F2α derivative-containing product of the present invention, the fluorine-containing prostaglandin exists in a state of being dissolved in water. The concentration of the fluorine-containing prostaglandin in the aqueous liquid preparation can be selected appropriately in view of the application of the aqueous liquid preparation. For example, in the case of an eye drop, the concentration of the fluorine-containing prostaglandin in the eye drop is preferably in the range of from 0.00005 to 0.05%, although it can be selected appropriately according to the disease to be treated or symptoms. Further, in the case where the nonionic surfactant is added to an eye drop, the amount of the nonionic surfactant added can also be appropriately increased or decreased according to the concentration of the fluorine-containing prostaglandin. In view of improvement of the solubility in water of the fluorine-containing prostaglandin, the concentration of the nonionic surfactant is preferably 5 times or more the concentration of the fluorine-containing prostaglandin. Moreover, when the solubility in water of the fluorine-containing prostaglandin is required to be further improved, the concentration of the nonionic surfactant is particularly preferably 10 times or more the concentration of the fluorine-containing prostaglandin.

When the prostaglandin F2 α derivative-containing product of the present invention is an eye drop, various pharmaceutically acceptable additives including an antioxidant such as ethylenediamine tetraacetic acid or dibutyl hydroxytoluene, a tonicity agent such as sodium chloride, potassium chloride, calcium chloride, glycerin or propylene glycol, a buffer such as boric acid, borax, citric acid, disodium hydrogenphosphate or ε-aminocaproic acid, a preservative such as benzalkonium chloride, chlorhexidine gluconate, benzethonium chloride, sorbic acid, potassium sorbate, ethyl parahydroxybenzoate or butyl parahydroxybenzoate can be added thereto in addition to the nonionic surfactant. A method for preparing the eye drop containing the fluorine-containing prostaglandin does not require a special method or process, and the eye drop can be prepared by a widely used method. Further, the pH of the eye drop is preferably adjusted to 3 to 8, particularly preferably 4 to 7.

As will be explained in detail in the section of storage stability test, when the aqueous liquid preparation containing the fluorine-containing prostaglandin is stored in the resin container made of a propylene/ethylene copolymer in which the ratio of a propylene component to an ethylene component is in the range of from 96.4/3.6 to 98.7/1.3 (propylene component/ethylene component), the prostaglandin is more stabilized than when it is stored in a resin container made of a propylene homopolymer and a decrease in the content of the fluorine-containing prostaglandin can be significantly inhibited.

Hereinafter, the present invention will be described in detail through carrying out a storage stability test (40°C for 7 days). However, such descriptions are disclosed for the purpose of understanding the present invention better and are not meant to limit the scope of the present invention.

### Example

### [Storage Stability Test]

### (1) Preparation of Eye Drop

As a typical example of a prostaglandin F2α derivative having at least a fluorine atom in its molecule, 16-phenoxy-15-deoxy-15,15-difluoro-17,18,19,20-tetranor-prostaglandin F2α isopropyl ester (hereinafter, referred to as the "present compound") was used. The present compound (0.0005%) and polysorbate 80 (0.05%) were dissolved in purified water, and then, commonly used additives such as disodium edetate (q.s.), concentrated glycerin (q.s.), benzalkonium chloride (q.s.) and the like were added thereto to obtain an eye drop with an osmotic pressure of about 1 and a pH of about 6.

### (2) Production of Resin Container

Resin containers of the present invention were obtained by injection blow molding of propylene/ethylene random copolymers (the ratios of a propylene component to an ethylene component are 98.7/1.3, 97.4/2.6 and 96.4/3.6 (propylene component/ethylene component)), and a propylene homopolymer, respectively.

### (3) Test Method

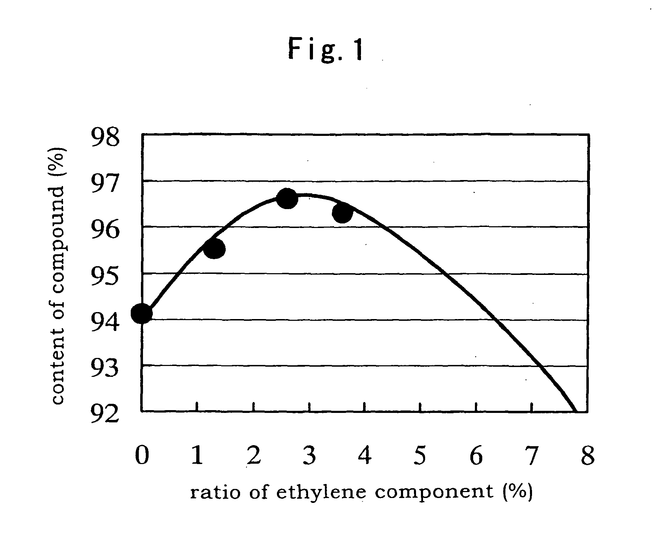

The eye drop (110 ml) obtained in the section of "(1) Preparation of Eye Drop" was placed in a glass container and the glass container was heated to 40°C. Then, the resin containers (7 containers each) obtained in the section of "(2) Production of Resin Container" were dipped in the glass container, and stored at a temperature of 40°C for 7 days. The amount of the present compound contained in the glass container was measured by high performance liquid chromatography before and after the storage, and the content of the present compound (average) was calculated taking the content of the present compound at the time of initiation of storage as 100%. The test results are shown in Fig. 1.

### (4) Discussion

As is clear from Fig. 1, when the present compound is stored in a container made of a propylene/ethylene random copolymer with each of the above-mentioned component ratios, the content of the present compound is higher than when it is stored in a container made of a propylene homopolymer, and the former container is excellent in the storage stability of the present compound.

[Fig. 1] Fig. 1 is a graph showing the results of Example and shows the relationship between the ratio of the ethylene component in a container made of a propylene/ethylene random copolymer with each component ratio and a container made of a propylene homopolymer and the content of the present compound.

## Claims

1. A prostaglandin F2α derivative-containing product, wherein an aqueous liquid preparation containing a prostaglandin F2α derivative having at least a fluorine atom in its molecule is stored in a resin container made of a propylene/ethylene copolymer in which the ratio of a propylene component to an ethylene component is in the range of from 96.4/3.6 to 98.7/1.3 (propylene component/ethylene component), whereby a decrease in the content of the prostaglandin F2α derivative in the aqueous liquid preparation is inhibited.

2. The prostaglandin F2α derivative-containing product according to claim 1, wherein the prostaglandin F2α derivative having at least a fluorine atom in its molecule is a difluoro-prostaglandin F2α derivative.

3. The prostaglandin F2α derivative-containing product according to claim 1, **characterized in that** a nonionic surfactant is contained in the aqueous liquid preparation.

4. The prostaglandin F2α derivative-containing product according to claim 3, wherein the nonionic surfactant is polysorbate 80.

5. The prostaglandin F2α derivative-containing product according to any one of claims 1 to 4, wherein the aqueous liquid preparation is an eye drop.

6. A method of inhibiting a decrease in the content of a prostaglandin F2α derivative having at least a fluorine atom in its molecule in an aqueous liquid preparation, comprising storing the aqueous liquid preparation containing the prostaglandin F2α derivative in a resin container made of a propylene/ethylene copolymer in which the ratio of a propylene component to an ethylene component is in the range of from 96.4/63.6 to 98.7/1.3 (propylene component/ethylene component).

## Patentansprüche

1. Prostaglandin-F2α-Derivat-enthaltendes Produkt, wobei eine wässrige flüssige Zubereitung, enthaltend ein Prostaglandin-F2α-Derivat, das mindestens ein Fluoratom in seinem Molekül aufweist, in einem Harzbehälter aufbewahrt wird, der aus einem Propylen/Ethylen-Copolymer hergestellt ist, wobei das Verhältnis eines Propylenbestandteils zu einem Ethylenbestandteil im Bereich von 96,4/3,6 bis 98,7/1,3 (Propylenbestandteil/Ethylenbestandteil) liegt, wodurch eine Abnahme im Gehalt des Prostaglandin-F2α-Derivats in der wässrigen flüssigen Zubereitung verhindert wird.

2. Prostaglandin-F2α-Derivat-enthaltendes Produkt nach Anspruch 1, wobei das Prostaglandin-F2α-Derivat, das mindestens ein Fluoratom in seinem Molekül aufweist, ein Difluor-Prostaglandin-F2α-Derivat ist.

3. Prostaglandin-F2α-Derivat-enthaltendes Produkt nach Anspruch 1, **dadurch gekennzeichnet, daß** ein nicht-ionisches grenzflächenaktives Mittel in der wässrigen flüssigen Zubereitung enthalten ist.

4. Prostaglandin-F2α-Derivat-enthaltendes Produkt nach Anspruch 3, wobei das nicht-ionische grenzflächenaktive Mittel Polysorbat-80 ist.

5. Prostaglandin-F2α-Derivat-enthaltendes Produkt nach einem der Ansprüche 1 bis 4, wobei die wässrige flüssige Zubereitung Augentropfen sind.

6. Verfahren zum Verhindern einer Abnahme im Gehalt eines Prostaglandin-F2α-Derivats, das mindestens ein Fluoratom in seinem Molekül aufweist, in einer wässrigen flüssigen Zubereitung, umfassend das Aufbewahren der wässrigen flüssigen Zubereitung, enthaltend das Prostaglandin-F2α-Derivat, in einem Harzbehälter, der aus einem Propylen/Ethylen-Copolymer hergestellt ist, wobei das Verhältnis eines Propylenbestandteils zu einem Ethylenbestandteil im Bereich von 96,4/3,6 bis 98,7/1,3 (Propylenbestandteil/Ethylenbestandteil) liegt.

## Revendications

1. Produit contenant un dérivé de la prostaglandine F2α, dans lequel une préparation liquide aqueuse contenant un dérivé de la prostaglandine F2α ayant au moins un atome de fluor dans sa molécule est stockée dans un contenant en résine constitué d'un copolymère propylène/éthylène, où le rapport du composant propylène au composant éthylène se situe dans l'intervalle allant de 96,4/3,6 à 98,7/1,3 (composant propylène/composant éthylène), où une diminution de la teneur en dérivé de la prostaglandine F2α dans la préparation liquide aqueuse est inhibée.

2. Produit contenant un dérivé de la prostaglandine F2α selon la revendication 1, où le dérivé de la prostaglandine F2α ayant au moins un atome de fluor dans sa molécule est un dérivé difluoroprostaglandine F2α.

3. Produit contenant un dérivé de la prostaglandine F2α selon la revendication 1, **caractérisé en ce qu'**un tensioactif non ionique est présent dans la préparation liquide aqueuse.

4. Produit contenant un dérivé de la prostaglandine F2α selon la revendication 3, où le tensioactif non ionique est le polysorbate 80.

5. Produit contenant un dérivé de la prostaglandine F2α selon l'une quelconque des revendications 1 à 4, où la préparation liquide aqueuse est des gouttes oculaires.

6. Procédé d'inhibition de la diminution de la teneur en dérivé de la prostaglandine F2α ayant au moins un atome de fluor dans sa molécule dans une préparation liquide aqueuse, comprenant le stockage de la préparation liquide aqueuse contenant le dérivé de la prostaglandine F2α dans un contenant en résine constitué d'un copolymère propylène/éthylène, où le rapport du composant propylène au composant éthylène se situe dans l'intervalle allant de 96,4/3,6 à 98,7/1,3 (composant propylène/composant éthylène).
